(19) Europäisches Patentamt / European Patent Office / Office européen des brevets

(11) EP 1 931 997 B1

# (12) FASCICULE DE BREVET EUROPEEN

(45) Date de publication et mention de la délivrance du brevet:
**03.05.2017 Bulletin 2017/18**

(21) Numéro de dépôt: **06808261.9**

(22) Date de dépôt: **29.08.2006**

(51) Int Cl.:
***G01N 33/542*** $^{(2006.01)}$ ***G01N 33/68*** $^{(2006.01)}$

(86) Numéro de dépôt international:
**PCT/FR2006/050821**

(87) Numéro de publication internationale:
**WO 2007/026099 (08.03.2007 Gazette 2007/10)**

## (54) PROCEDE POUR LA MISE EN EVIDENCE D'UN PROCESSUS BIOLOGIQUE PAR MESURE D'UN FRET

VERFAHREN ZUR UNTERSUCHUNG EINES BIOLOGISCHEN VORGANGS UNTER VERWENDUNG EINES FRET-VERFAHRENS

METHOD OF EXAMINING A BIOLOGICAL PROCESS USING A FRET MEASUREMENT

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **30.08.2005 FR 0508857**

(43) Date de publication de la demande:
**18.06.2008 Bulletin 2008/25**

(73) Titulaire: **CISBIO BIOASSAYS**
**30200 Codolet (FR)**

(72) Inventeurs:
- **TRINQUET, Eric**
  **30130 Pont Saint Esprit (FR)**
- **ANSANAY, Hervé**
  **34000 Montpellier (FR)**
- **MATHIS, Gérard**
  **30200 Bagnols sur Cèze (FR)**

(74) Mandataire: **Nevant, Marc et al**
**Cabinet Beau de Loménie**
**158, rue de l'Université**
**75340 Paris Cedex 07 (FR)**

(56) Documents cités:
- **SORAGNA A ET AL: "Functionally independent subunits in the oligomeric structure of the GABA cotransporter rGAT1." CELLULAR AND MOLECULAR LIFE SCIENCES : CMLS. DEC 2005, vol. 62, no. 23, décembre 2005 (2005-12), pages 2877-2885, XP002371705 ISSN: 1420-682X**
- **MAUREL DAMIEN ET AL: "Cell surface detection of membrane protein interaction with homogeneous time-resolved fluorescence resonance energy transfer technology." ANALYTICAL BIOCHEMISTRY. 15 JUN 2004, vol. 329, no. 2, 15 juin 2004 (2004-06-15), pages 253-262, XP002371706 ISSN: 0003-2697**
- **SHIVERS B D ET AL: "Two novel GABAA receptor subunits exist in distinct neuronal subpopulations." NEURON. SEP 1989, vol. 3, no. 3, septembre 1989 (1989-09), pages 327-337, XP002371707 ISSN: 0896-6273**
- **GUIGNET EMMANUEL G ET AL: "Reversible site-selective labeling of membrane proteins in live cells." NATURE BIOTECHNOLOGY. APR 2004, vol. 22, no. 4, avril 2004 (2004-04), pages 440-444, XP002371708 ISSN: 1087-0156**
- **TURCATTI G ET AL: "Probing the structure and function of the tachykinin neurokinin-2 receptor through biosynthetic incorporation of fluorescent amino acids at specific sites." THE JOURNAL OF BIOLOGICAL CHEMISTRY. 16 AUG 1996, vol. 271, no. 33, 16 août 1996 (1996-08-16), pages 19991-19998, XP002371709 ISSN: 0021-9258**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 931 997 B1

## Description

### Domaine de l'invention

La présente invention concerne le domaine de la biologie cellulaire et plus particulièrement, l'étude des interactions entre des entités biologiques ou entre les différentes parties constitutives de ces entités, éventuellement en présence de ligands potentiels, afin d'élucider les processus biologiques ou pathologiques et de cribler de nouveaux médicaments.

On tente de plus en plus d'étudier les processus biologiques ou pathologiques au sein de préparations membranaires ou de cellules vivantes et on utilise fréquemment à cet effet des tests de mesure de fluorescence, en particulier les tests de fluorescence dans lesquels on mesure le transfert d'énergie non radiatif de résonance entre un fluorophore donneur et un fluorophore accepteur.

Après excitation lumineuse à la longueur d'onde d'excitation du fluorophore donneur, un transfert d'énergie a lieu entre le fluorophore donneur et le fluorophore accepteur s'ils sont proches l'un de l'autre ; ce transfert d'énergie se caractérise par une émission de lumière par l'accepteur (signal de FRET) qui peut être mesurée à l'aide d'un fluorimètre.

L'utilisation de chélates ou de cryptates de terres rares comme fluorophores donneurs, a permis de développer la technique connue sous le nom de HTRF® (Homogenous Time Resolved Fluorescence) (voir notamment « Homogeneous time resolved fluorescence energy transfer using rare earth cryptates as a tool for probing molecular interactions in biology », Spectrochimica Acta Part A 57 (2001) 2197-2211) ; cette technique présente de nombreux avantages qui ont déjà permis plusieurs applications dans le domaine du diagnostic in vitro et dans celui du criblage à haut débit dans l'industrie pharmaceutique.

### Etat de la technique

La technologie du FRET en temps résolu pour la détection à la surface des cellules des interactions entre des protéines membranaires a été décrite par Damien Maurel et al. (voir document 1). J.P. Pin et al. et Jianfeng Liu et al. ont étudié le récepteur $GABA_B$ par la technologie de FRET en temps résolu (voir documents 2 et 3).

Dans tous ces travaux, les membres du couple de partenaires de FRET sont situés dans le milieu extracellulaire.

Il a été proposé d'étudier les canaux ioniques qui sont des cibles importantes pour les médicaments par un système de FRET (voir les documents 4, 5 et 6). Dans ces études, le fluorophore donneur est généralement la coumarine liée à une chaîne lipidique, qui est fixé sur la partie extracellulaire de la membrane plasmique des cellules et l'accepteur est un colorant de type oxonol qui peut migrer à l'intérieur de la membrane plasmique en fonction du potentiel membranaire. Dans ces études, les fluorophores ne sont donc jamais situés de part et d'autre de la membrane plasmique.

Turcatti et al. et Chollet et al. (voir documents 7 et 8) ont étudié le récepteur $NK_2$ par un système de FRET en utilisant des préparations membranaires d'oocytes dans lequel le fluorophore accepteur est un ligand couplé à de la rhodamine et le fluorophore donneur est le nitrobenzoxadiazole (NBD) incorporé de manière spécifique à des endroits précis du récepteur $NK_2$, situés de part et d'autre de la membrane plasmique.

F. Chan et al. (voir document 9) décrivent l'utilisation du FRET entre des variants de la protéine fluorescente verte (GFP) comme fluorophore donneur pour étudier les interactions entre récepteurs à la surface des cellules. Diverses constructions de récepteurs ont été réalisées avec la protéine fluorescente bleue (CFP) ou la protéine fluorescente jaune (YFP) comme fluorophore accepteur sur les parties extra-cellulaires ou intra-cellulaires.

Les expériences effectuées pour détecter l'association des récepteurs montrent qu'un FRET est détecté seulement lorsque le fluorophore accepteur et le fluorophore donneur sont sur la partie intracellulaire ou la partie extracellulaire du récepteur. En revanche, aucun FRET n'a pu être détecté lorsque l'un des fluorophores est sur la partie intracellulaire et l'autre fluorophore est sur la partie extracellulaire du récepteur, c'est-à-dire lorsque les fluorophores sont de part et d'autre de la membrane plasmique (voir figure 4 de ce document).

L'équipe de M.C. Wilson et al. (voir document 10) a également constaté, lors de l'étude de l'interaction entre le transporteur lactate MCT1 et la glycoprotéine CD147 à la surface de cellules COS, qu'un FRET n'est détectable que lorsque le donneur et l'accepteur sont situés dans la partie intracellulaire de MCT1 et CD147. En revanche, aucune des constructions où le fluorophore donneur et le fluorophore accepteur sont de part et d'autre de la membrane plasmique ne donne de signal de FRET (voir tableau 1 de ce document).

Ces systèmes de FRET ne permettent donc pas de détecter les interactions entre les entités biologiques lorsqu'elles sont de part et d'autre de la membrane plasmique. Or, on sait que les processus biologiques sont activés ou inhibés par le transfert d'informations de l'extérieur de la cellule vers l'intérieur de la cellule.

Il s'avère donc intéressant et hautement souhaitable de pouvoir détecter directement les interactions provoquées par une entité biologique ou un ligand dans le milieu extracellulaire sur une entité biologique dans le milieu intracellulaire de cellules vivantes.

Il a été proposé par Guignet et al. (voir document 11) de marquer sélectivement les protéines membranaires

dans les cellules vivantes, en utilisant un fluorophore donneur, en l'occurrence une protéine fluorescente verte, et un accepteur non fluorescent, et de mesurer la disparition de la fluorescence du donneur, les donneur et accepteur pouvant être de part et d'autre de la membrane plasmique.

Ce mode de mesure ne permet pas de faire la distinction entre une diminution de la fluorescence due au transfert d'énergie entre le donneur et l'accepteur et une diminution de la fluorescence due à des variations des propriétés optiques du milieu dans lequel le transfert d'énergie est mesuré.

Un tel système ne peut donc être utilisé que lorsque les variations optiques sont considérables.

WO 03/005028 décrit un procédé de sélection d'agents actifs basé sur la mesure de modifications de luminescence liées à des changements de distance entre deux luminophores, dont l'un est situé dans la membrane cellulaire tandis que l'autre est recruté ou non à la membrane cellulaire en fonction de son activité biologique. La variation de luminescence peut être déterminée en mesurant des variations de FRET entre les deux luminophores. Les luminophores utilisés dans WO 03/005028 sont d'une part, un agent intermédiaire fluorescent ou luminescent produit par le système cellulaire, et d'autre part un luminophore lipophile compatible avec les membranes cellulaires naturelles et réalisant un couplage de fluorescence avec ledit agent intermédiaire. WO 03/005028 ne décrit pas de systèmes mettant en jeu un transfert d'énergie transmembranaire puisque l'un des luminophores est localisé à l'intérieur de la membrane plasmique, soit dans son feuillet externe soit dans son feuillet interne. Ainsi, le procédé décrit dans cette demande ne permet pas d'étudier des phénomènes biologiques qui auraient pour conséquence un transfert d'énergie totalement transmembranaire, c'est-à-dire traversant les deux feuillets interne et externe de la bicouche lipidique. De plus, dans la mesure où l'un des luminophores est un lipide ou est couplé à un lipide distribué de manière homogène dans la membrane plasmique, ce procédé ne permet pas d'étudier des phénomènes biologiques spécifiques.

Il existe donc un réel besoin de moyens pour mettre en évidence les processus biologiques qui résultent de l'interaction entre des molécules biologiques ou des parties constitutives de ces molécules lorsque celles-ci sont dans un milieu de mesure contenant une membrane plasmique ou toute autre membrane lipidique.

## Résumé de l'invention

On a maintenant trouvé qu'il est possible de mettre en évidence un processus biologique dans un système cellulaire par mesure d'un FRET en utilisant comme fluorophore donneur un filuorophore ayant une durée de vie longue, les fluorophores donneur et accepteur étant couplés sur des entités biologiques et lesdits fluorophores donneur et accepteur étant situés de part et d'autre d'une membrane lipidique.

Ainsi, la présente invention a pour objet un procédé pour la mise en évidence d'un processus biologique par mesure d'un FRET qui consiste à :

- incorporer dans un milieu de mesure contenant une membrane lipidique une entité biologique X couplée avec un premier membre d'un couple de partenaires de FRET et une deuxième entité biologique Y couplée avec le deuxième membre du couple de partenaires de FRET, ledit couple de partenaires de FRET étant composé d'un fluorophore donneur et d'un fluorophore accepteur, le fluorophore donneur ayant une durée de vie comprise entre 100ns et 5000$\mu$s, de préférence comprise entre 100 ns et 3000$\mu$s, et les membres dudit couple de partenaires de FRET étant situés de part et d'autre de la membrane lipidique ;
- exciter le milieu de mesure à la longueur d'onde d'excitation du membre donneur d'énergie ;
- mesurer le signal de FRET ou les variations dudit signal émis dans ledit milieu de culture.

Selon une variante, le procédé selon l'invention comprend une étape de stimulation du milieu de mesure, ladite stimulation pouvant être une stimulation électrique, mécanique ou thermique. Il est en effet connu que de telles stimulations peuvent se traduire au niveau cellulaire par l'activation de processus biologiques. Ainsi, un changement de température du milieu peut provoquer des modifications de la conformation de certaines protéines transmembranaires, comme les thermorécepteurs, qui peuvent être étudiées grâce au processus selon l'invention. De même, certaines voies de signalisation peuvent être déclenchées par l'activation de mécanorécepteurs, ou encore par des variations du potentiel membranaire. Le marquage sélectif des entités biologiques X et Y impliquées dans ces processus et la mesure des variations de FRET transmembranaire après stimulation permet d'étudier ces voies de signalisation.

Selon une autre variante, le procédé de l'invention comprend une étape de stimulation chimique ; dans ce cas, le processus selon l'invention est mis en oeuvre en présence d'un composé dont on veut tester l'effet sur les cellules, par exemple s'il est agoniste ou antagoniste vis-à-vis d'un récepteur membranaire constitué par l'une des entités biologiques X ou Y. Le procédé selon l'invention est donc très utile pour cribler des banques de composés à tester, ces composés étant par exemple issus de synthèses par chimie combinatoire. En effet, la mesure de la variation du FRET transmembranaire entre les entités biologiques X et Y impliquées dans un processus biologique donné, en présence ou en l'absence du composé à tester, permet de mettre en évidence un éventuel effet biologique du composé à tester sur le processus biologique étudié.

Les processus biologiques qui peuvent être mis en évidence par le procédé de l'invention sont nombreux et diffèrent selon la nature des entités biologiques mises en oeuvre.
Ces processus biologiques sont par exemple :

- les phénomènes de dimérisation de deux protéines intrinsèques, par exemple l'homodimérisation ou l'hétérodimérisation de récepteurs transmembranaires ;
- le phénomène de translocation d'un composé cytosoluble vers ou à partir d'une protéine membranaire ;
- la détection de ligands spécifiques de récepteurs membranaires et le criblage de médicaments ;
- les changements de structure tridimensionnelle d'un récepteur transmembranaire en présence d'un ligand, ou encore les changements de structure de complexes protéiques.

Le couplage des entités biologiques avec l'un des membres d'un couple de partenaires de FRET est réalisé soit directement par une ou plusieurs liaisons covalentes selon des techniques bien connues de l'homme du métier (Bioconjugate Techniques, G.T. Hermanson, Academic Press, 1996), soit indirectement par l'intermédiaire de partenaires de liaison de type étiquette ("tag") / anticorps anti-étiquette (anti-tag), antigène / anticorps, avidine ou streptavidine / biotine ; haptène / anticorps.
La mesure du signal de FRET est effectuée selon les méthodes classiques bien connues de l'homme du métier et décrites par exemple, par Mathis G., " Rare Earth Cryptates and Homogeneous Fluoroimmunoassays with Human Sera. " Clin Chem 1993, 39, n° 9, 1953-1959.
Par exemple, les variations du signal de FRET peuvent être mesurées de manière quantitative par des détecteurs de fluorescence classiques couramment utilisés, par l'homme du métier, dans les laboratoires spécialisés dans le criblage à haut débit (par exemple avec le fluorimètre Rubystar de la société BMG labs).
La mesure des variations du signal de FRET permet de rendre compte directement d'un processus biologique et présente une solution adaptée à la recherche de molécules modulant ces processus dans un criblage à haut débit.

<u>Description détaillée de l'invention</u>

1) <u>Le milieu de mesure</u>

Le milieu de mesure est constitué d'un milieu biologique contenant une membrane lipidique.
La plupart du temps, ce milieu biologique est une culture de cellules, c'est-à-dire contenant des cellules vivantes dans un milieu de culture. De manière plus large, on entend par milieu biologique toute préparation comprenant des tissus cellulaires, des cellules vivantes ou non, des lysats de cellules, ou encore des systèmes biologiques reconstitués comprenant les protéines nécessaires à l'étude d'un processus biochimique donné.
Par "membrane lipidique" on désigne dans la présente description : la membrane plasmique, la membrane du réticulum endoplasmique, la bi-membrane mitochondriale, une membrane lysosomiale, la membrane nucléaire, la membrane d'une vésicule golgienne. Avantageusement, la membrane lipidique est la membrane plasmique et le milieu de mesure, une préparation de cellules transfectées.
Dans la présente description, on désigne par "préparation de cellules transfectées", les cellules transfectées elles-mêmes, les cellules transfectées perméabilisées par des techniques bien connues de l'homme du métier, notamment mettant en oeuvre des détergents tel que le Triton X100 ainsi que les préparations membranaires de cellules transfectées.
Les cellules peuvent être transfectées de manière stable, c'est-à-dire que les séquences codant pour les entités biologiques X et Y sont intégrées dans l'ADN des cellules.
Les cellules peuvent également être transfectées de manière transitoire à l'aide d'un vecteur d'expression de type plasmide contenant la séquence d'acide nucléique codant pour lesdites entités biologiques X et Y.
Les techniques de transfection des cellules, bien connues de l'homme du métier, sont décrites par exemple dans l'ouvrage de Sambrook et al., Molecular Cloning : A Laboratory Manual ; 2nd edition ; Cold Spring Harbor Laboratory, Cold Spring Harbor, N.Y. (1989) ainsi que par Mehier-Humbert S, Guy RH "Advanced Drug Delivery Reviews" (2005) Physical methods for gene transfer: improving the kinetics of gene delivery into cells, 57 (5) p 733-753. De plus, les transfections dans des cellules mammifères, sont décrites dans "Methods" Volume 33, Issue 2, p 93-186 (Juin 2004).
Les cellules utilisées sont généralement des cellules de mammifères, telles que par exemple les cellules HEK 293.
Les entités biologiques X et Y peuvent également être incorporées dans les cellules par perméabilisation de celles-ci, par exemple par traitement ménagé avec un détergent tel que le Triton X100.

2) Les entités biologiques

Les entités biologiques X et Y, sont choisies parmi les protéines membranaires intrinsèques ou extrinsèques, les protéines transmembranaires, telles que les récepteurs transmembranaires, des composés cytosolubles, les composés organiques ou biologiques faisant partie d'une banque de composés dont on veut tester l'effet sur des cellules, désignés ci-après par le terme "ligands", des anticorps.

Des exemples de protéines transmembranaires sont les récepteurs, tels que notamment les récepteurs à sept domaines transmembranaires couplés aux protéines G (ci-après dénommés RCPG), qui sont impliqués dans de nombreux processus pathologiques. En effet, les RCPG sont des protéines transmembranaires responsables de la reconnaissance et du transfert de l'information de l'extérieur vers l'intérieur de la cellule. Ces protéines représentent des cibles thérapeutiques importantes et plus de 50 % des médicaments actuels ciblent ces récepteurs ou leur cascade de transduction.

Par "composé membranaire intrinsèque", on désigne les composés qui sont situés dans une membrane lipidique, par exemple la membrane plasmique. A titre d'exemple de composés membranaires intrinsèques, on peut citer les protéines transmembranaires, les récepteurs transmembranaires, tels que les RCPG, les proteines canaux, les protéines de transport ou d'échange, les protéines qui stabilisent la structure de la membrane plasmique, les immunoglobulines, les récepteurs hormonaux, les récepteurs à activité tyrosine kinase, les enzymes comme l'adénylyl cyclase.

Par "composé membranaire extrinsèque", on désigne les composés qui sont situés d'un côté ou de l'autre d'une membrane lipidique. On peut citer à titre d'exemple des protéines d'ancrage situées sur la face externe de la membrane plasmique, des enzymes situées sur la face interne de la membrane plasmique comme les phospholipases, des protéines impliquées dans les processus de transduction du signal comme les protéines G hétérotrimériques, les kinases des récepteurs couplés aux protéines G (GRK), les arrestines, les protéines interagissant avec les arrestines pour constituer des échafaudages sous-membranaires comme les protéines du trafic [Clathrine, AP2 (protéine d'adaptation 2), NSF (facteur d'échange nucléotidique pour ARF, ARF étant le facteur d'ADP-ribosylation], les petites protéines G et facteurs d'échange nucléotidique (ARF6, ARNO, RhoA...), les protéines de signalisation (les MAP-kinases, MAP désignant les protéines mitogéniques activées, c-Src, Yes, Mdm2...) les phosphodiestérases (PDE4...), les protéines phosphatases (PP2A...)

3) Les membres du couple de partenaires de FRET

Le couple de partenaires de FRET comprend un fluorophore donneur et un fluorophore accepteur, l'un des fluorophores étant un fluorophore à durée de vie longue, c'est-à-dire un fluorophore ayant une durée de vie supérieure à 100 ns, de préférence comprise entre 100 ns et 5000 μs, de préférence encore entre 100 et 3000 μs.

Des exemples de fluorophores à durée de vie longue qui conviennent aux fins de l'invention sont les complexes de terres rares, en particulier les complexes de terbium et d'europium.

Les complexes de terre rare sont des composés connus qui sont décrits par exemple dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. D'autres chélates sont composés d'un ligand nonadenté tel que la terpyridine (voir EP 403 593, US 5 324 825, US 5 202 423, US 5 316 909).

Avantageusement, le complexe de terre rare est un chélate ou un cryptate. Lorsque la terre rare est l'europium, le complexe de terre rare est de préférence un cryptate de terre rare à motif pyridine, de manière encore plus préférée à motif pyridine-bipyridine.

Les cryptates de terres rares sont décrits dans les brevets EP 0 180 492, EP 0 601 113 et la demande WO 01/96 877.

Parmi ces cryptates, on préfère tout particulièrement les cryptates à motif pyridine-bipyridine de formule :

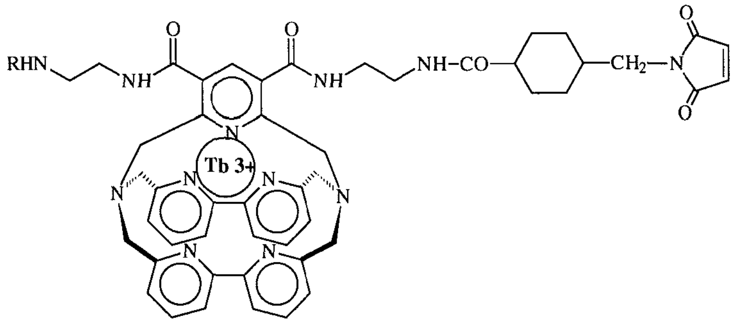

L'autre fluorophore membre du couple de partenaires de FRET est une substance fluorescente choisie parmi les rhodamines, les cyanines, les squaraines, les fluorophores connus sous la dénomination BODIPYs (difluoroboradiazaindacènes), les fluorescéines, les fluorophores connus sous la dénomination AlexaFluor, les quantum dots, les protéines fluorescentes comme la protéine fluorescente verte (GFP) ou ses variants, les protéines fluorescentes extraites de coraux, les phycobiliprotéines, telles que la B-phycoérythrine, la R-phycoérythrine, la C-phycocyanine, les allophycocyanines, en particulier celles connues sous la dénomination XL665.

La sélection des membres d'un couple de partenaires de FRET est à la portée de l'homme du métier. A cet effet, il pourra se référer à l'ouvrage de Lakowicz, Principles of fluorescence spectroscopy, 2nd edition,Kluwer academic/plenum publishers, NY (1999).

Par "couple fluorophore donneur / fluorophore accepteur", on entend dans la présente description, les couples de fluorophores dont le spectre d'absorption de l'accepteur recouvre au moins partiellement le spectre d'émission du donneur de telle manière qu'une compatibilité énergétique existe entre les deux molécules. De préférence, cette compatibilité énergétique se traduit par un rayon de Förster (distance donneur/accepteur pour laquelle l'efficacité de transfert est égale à 50%) supérieur à 4 nm.

Des couples de partenaires de FRET particulièrement appropriés aux fins de l'invention sont les couples suivants dont le donneur est un cryptate de terbium, et l'accepteur est une protéine fluorescente, telle que l'YFP. D'autres couples de partenaires de FRET appropriés aux fins de l'invention sont les couples dont le donneur est un cryptate d'europium et l'accepteur est soit de l'allophycocyanine réticulée, connue sous la dénomination commerciale XL 665, le fluorophore d2 commercialisé par CIS bio international, ou encore l'AlexaFluor 647.

4) <u>Le couplage des membres du couple de partenaires de FRET (fluorophores)</u>.

Comme indiqué précédemment, le couplage des membres d'un couple de partenaires de FRET aux entités biologiques X ou Y est réalisé, soit directement par une ou plusieurs liaisons covalentes, soit indirectement par l'intermédiaire de partenaires de liaison de type étiquette ("tag") / anticorps anti-étiquette (anti-tag), antigène / anticorps, avidine ou streptavidine / biotine ; haptène / anticorps.

Le couplage direct des fluorophores peut être réalisé :

1) par expression d'une protéine de fusion entre lesdites entités biologiques X ou Y et une protéine fluorescente ; ou
2) par expression d'une protéine de fusion entre lesdites entités biologiques X ou Y et une protéine ayant une activité enzymatique irréversible (couramment dénommée enzyme suicide) qui transfère le fluorophore sur ladite entité biologique X ou Y ;
3) par épissage avec une intéine.

Cette technique de couplage par épissage avec une intéine est décrite par exemple dans The Journal of Biological Chemistry, vol. 273, n°26, pp 15887-15890, 26 juin 1998 et J. Am. Chem. Soc. 2003, 125, 7180-7181.

Le couplage indirect des fluorophores peut être réalisée :

1) par l'intermédiaire d'un couple "ligand-entité biologique" ou "tag / antitag".
2) par expression des entités biologiques X ou Y natives, dans ce cas les composés fluorescents sont conjugués à un anticorps reconnaissant spécifiquement les entités biologiques ; ou
3) par expression des entités biologiques couplées à une enzyme suicide qui transfère une étiquette sur lesdites entités biologiques.

Ces méthodes de couplage direct et indirect de fluorophores utilisent les techniques d'ADN recombinant bien connues de l'homme du métier.

Le couplage direct d'un fluorophore par expression d'une protéine de fusion entre lesdites entités biologiques X ou Y et une protéine ayant une activité enzymatique irréversible utilise avantageusement, comme protéine à activité enzymatique irréversible, une $O^6$-alkylguanine-DNA alkyl transférase (AGT) (voir WO 02/083937) ou une déhalogénase.

Dans ce cas, les cellules sont transfectées avec un acide nucléique codant pour l'une desdites entités biologiques et avec un acide nucléique codant pour ladite protéine à activité enzymatique irréversible. Elles sont ensuite mises en présence du substrat spécifique de ladite protéine à activité enzymatique, qui porte le fluorophore que l'on veut coupler au récepteur ou à l'une des sous-unités $G\alpha$ ou $G\beta y$.

Dans le couplage indirect d'un fluorophore par l'intermédiaire d'un couple "ligand-récepteur" ou "tag / antitag", on fait exprimer une protéine de fusion entre l'une desdites entités biologiques et une séquence peptidique dite "étiquette" ou "tag", les composés fluorescents étant dans ce cas conjugués à un anticorps reconnaissant spécifiquement l'étiquette. On utilise les séquences peptidiques couramment utilisées en biologie moléculaire, telles que par exemple les étiquettes "Myc" ou "FLAG" mentionnées ci-après.

Le terme "couple ligand-récepteur" désigne deux partenaires de liaison tels que les couples : haptène/anticorps ; DNP/ anticorps anti-DNP, dans lequel DNP représente le dinitrophénol ; GST/anticorps anti-GST dans lequel GST représente la glutathion S-transférase ; biotine/avidine ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ; FLAG®/anticorps anti-FLAG ® dans lequel FLAG ® est un peptide ayant les 8 acides aminés ci-après DYKDDDDK ; HA/anticorps anti HA dans lequel HA est un épitope de l'hémagglutinine d'Influenza, constitué des 9 acides aminés ci-après : YPYDVPDYA.

Ces couples couramment dénommés en langue anglaise "tag/antitag" sont bien connus de l'homme du métier et sont disponibles commercialement.

5) La mesure du signal de FRET

Le signal de FRET mesuré peut être directement corrélé au phénomène biologique étudié : En effet, le niveau du transfert d'énergie entre le composé fluorescent donneur et le composé fluorescent accepteur est proportionnel à l'inverse de la distance entre ces composés à la puissance 6. Pour les couples donneurs-accepteurs communément utilisés par l'homme du métier, la distance Ro (correspondant à une efficacité de transfert de 50%) est de l'ordre du nanomètre, ce qui fait du FRET un outil de choix pour l'étude des interactions biologiques.

La variation du signal de FRET est fonction du type de phénomène biologique, mais de manière générale, un rapprochement des composés fluorescents donneurs et accepteurs provoque un transfert d'énergie du composé donneur vers le composé accepteur, qui entraîne une diminution du signal de fluorescence émis par le composé donneur et une augmentation du signal émis par le composé accepteur, et inversement. La plupart des phénomènes biologiques impliquant des interactions entre différents partenaires vont donc pouvoir être étudiés en mesurant l'évolution du FRET entre 2 composés fluorescents couplés à des composés qui vont être plus ou moins éloignés selon le phénomène biologique mis en oeuvre.

Le signal de FRET peut être mesuré de différentes façons : mesure de la fluorescence émise par le donneur seul, par l'accepteur seul, par le donneur et l'accepteur ou encore mesure de la variation de la polarisation de la lumière émise dans le milieu par l'accepteur suite au FRET. On peut aussi inclure la mesure du FRET en observant la variation de durée de vie au niveau du donneur ce qui est facilité par l'utilisation d'un donneur à durée de vie de fluorescence longue tel que les complexes de terre rare (notamment sur de l'appareillage simple comme des lecteurs de plaques).

Par ailleurs, le signal de FRET peut être mesuré à un instant précis ou à intervalles réguliers, ce qui permet d'étudier son évolution au cours du temps et procéder ainsi à des études de cinétique du processus biologique étudié.

6) Les processus biologiques

Selon la nature des entités biologiques mises en oeuvre, le procédé de l'invention permet de détecter différents processus biologiques qui sont explicités ci-après. Dans tous les cas, ces processus sont étudiés en mesurant un FRET transmembranaire entre 2 entités biologiques impliquées dans lesdits processus.

a) modifications de la structure tridimensionnelle d'un récepteur transmembranaire en présence d'un ligand à tester.

Il est connu que la structure tridimensionnelle des protéines peut varier lors de phénomènes biologiques, comme par exemple la liaison d'un ligand à son récepteur. On peut citer tout particulièrement les variations de structure de protéines transmembranaires en réponse à la liaison avec leur ligand, comme c'est par example le cas pour le récepteur GABA, le récepteur du glutamate (GluR) ((Parmentier ML, Prezeau L, Bockaert J, Pin JP. (2002) A model for the functioning of family 3 GPCRs. Trends Pharmacol Sci. 23 (6): 268-74, Bissantz C. (2003) Conformational changes of G protein-coupled receptors during their activation by agonist binding. J Recept Signal Transduct Res. 23 (2-3): 123-53.).

Lorsque les entités biologiques X et Y sont un seul et même récepteur transmembranaire et la membrane lipidique est la membrane plasmique, la variation du signal de FRET en présence d'un ligand à tester sera significative d'une modification de la structure tridimensionnelle dudit récepteur provoquée par le ligand à tester.

La méthode selon l'invention peut également être mise en oeuvre pour étudier des variations de structures de complexes protéiques : c'est le cas par exemple des complexes RCP (receptor component protein)-protéines G : dans ce cas, le composé X est un RCP, le composé Y une protéine G , les composés X et Y formant un complexe protéique. Lors de l'activation du composé X, les composés X et Y continuent d'interagir mais les modifications structurales résultant de l'activation de X pourront être détectées en mesurant le signal de FRET. ( voir Prado MA, Evans-Bain B, Dickerson IM. (2002) Receptor component protein (RCP): a member of a multi-protein complex required for G-protein-coupled signal transduction Biochem Soc Trans. 30 (4): 460-4.

b) phénomène de dimérisation des protéines membranaires intrinsèques.

Dans le cas où les deux entités biologiques X et Y sont deux protéines transmembranaires et la membrane lipidique est la membrane plasmique, la variation du signal de FRET en présence ou en l'absence d'un composé à tester sera indicative d'une interaction entre lesdites protéines.
De telles interactions peuvent être constitutives ou induites par un événement biologique.
Il est connu que le fonctionnement de certains récepteurs membranaires est modulé par leur dimérisation. Cette dimérisation peut être une homodimérisation dans le cas où les deux récepteurs sont identiques, ou une hétérodimérisation si les récepteurs sont différents. On peut citer à titre d'exemples de tels récepteurs à travers les trois familles connues de récepteurs : le récepteur $GABA_B$, le récepteur à l'EGF, les récepteurs métabotropiques du Glutamate (mGluRs), les récepteurs de la vasopressine, les récepteurs $\beta$-adrénergiques, les récepteurs aux opiacés, les récepteurs aux chemokines, les récepteurs de la mélatonine, les récepteurs aux hormones glycoprotéiques. Ce phénomène de dimérisation est connu de l'homme du métier et largement décrit dans la littérature (voir par exemple : Sebastien Bulenger, Stefano Marullo et Michel Bouvier (2005) Emerging role of homo- and hetero-dimerization in G-protein coupled receptor biosynthesis and maturation. Trends in Pharmacological Sciences, v26 (3) pp131-37 ; Hansen JL, Sheikh SP. (2004) Functional consequences of 7TM receptor dimerization. Eur J Pharm Sci. 23 (4-5): 301-17 ; Vassart G, Pardo L, Costagliola S. (2004) A molecular dissection of the glycoprotein hormone receptors. Trends Biochem Sci. 29 (3): 119-26 ; Milligan G. (2004) G protein-coupled receptor dimerization: function and ligand pharmacology. Mol Pharmacol. 66 (1): 1-7 ; Terrillon, S. and Bouvier, M. (2004) Roles of G-protein-coupled receptor dimerization. EMBO Rep. 5, 30-34 ; Rios CD, Jordan BA, Gomes I, Devi LA. (2001) G-protein-coupled receptor dimerization: modulation of receptor function. Pharmacol Ther. 92 (2-3): 71-87.

c) phénomène de translocation d'un composé cytosoluble vers la membrane plasmique.

Lorsque l'entité biologique X est un composé cytosoluble et l'entité Y est un composé membranaire intrinsèque ou extrinsèque, la variation du signal de FRET sera indicative d'un phénomène de translocation dudit composé cytosoluble vers ou à partir de ladite protéine membranaire.
On peut citer comme exemple de tels phénomènes la translocation de molécules d'arrestine vers la membrane plasmique, qui participe au phénomène d'internalisations de protéines transmembranaires, notamment de certains récepteurs couplés aux protéines G. On peut également citer les phénomènes de translocation des protéines G vers ou à partir de protéines membranaires, tels que les récepteurs couplés aux protéines G.

d) liaison d'un ligand à tester à un récepteur transmembranaire.

Si la membrane lipidique est la membrane plasmique et si l'entité biologique X est un récepteur transmembranaire situé sur la membrane plasmique et marqué dans sa partie intracellulaire par un membre d'un couple de composés fluorescents donneur / accepteur et l'entité biologique Y est un ligand potentiel dudit récepteur transmembranaire faisant partie d'une banque de composés à tester et marqué par l'autre membre du couple de composés fluorescents donneur / accepteur, la variation du signal de FRET sera indicative de la liaison dudit ligand au récepteur. Ce procédé est particulièrement avantageux pour cribler des banques de composés issus de la chimie combinatoire.
Le même type de procédé peut être mis en oeuvre par une méthode dite de compétition : dans ce cas, X est un récepteur transmembranaire situé sur la membrane plasmique et marqué dans sa partie intracellulaire par un membre d'un couple de composés fluorescents donneur / accepteur, Y est un ligand connu dudit récepteur transmembranaire, et marqué par l'autre membre du couple de composés fluorescents donneur / accepteur; un composé à tester est ajouté au milieu de mesure et l'on détermine la variation du signal de FRET en présence et en l'absence de ce composé. Si le composé à tester est effectivement capable de se lier au récepteur X, il rentre en compétition avec le ligand Y pour se lier au récepteur X, ce qui se traduit par une variation du signal de FRET transmembranaire. Ce procédé est également avantageusement utilisé pour procéder au criblage de banques de composés chimiques.
L'invention va être maintenant décrite plus en détail par les exemples illustratifs mais non limitatifs ci-après.

EXEMPLE 1 : Constructions moléculaires :

Deux constructions, qui sont schématisées sur la Figure 1, ont été utilisées :

- Construction 1 : HA-GABAB R-1 (" HA-GB1 ") + FLAG-GABAB R-2 (FLAG-GB2),
- Construction 2 : HA-GABAB R-1 (" HA-GB1 ") + FLAG-GABAB R-2 -YFP (FLAG-GB2-YFP).

EXEMPLE 2: réactifs

Dans les expériences décrites ci-dessous, les réactifs suivants sont utilisés :

- Un anticorps anti-FLAG marqué au cryptate de terbium suivant :

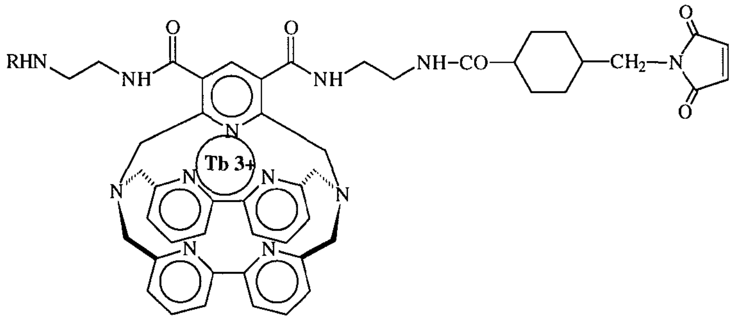

- Un anticorps anti-HA marqué à l'Alexa 647: Le marquage de l'anticorps anti-HA a été réalisé à l'aide de l'Alexa 647 succinimidyl ester (Molecular Probes ref A-2006). La réaction de marquage s'est faite dans un tampon carbonate 0.1M pH=9 durant 30 minutes à température ambiante avec un excès molaire de 4 Alexa647 par anticorps. L'excès de sonde fluorescente n'ayant pas réagi avec l'anticorps est éliminé par chromatographie d'exclusion (Gel Pharmacia Biotech G-25 super fine). Le taux de marquage final de l'anticorps, déterminé à l'aide du spectre d'absorption du conjugué, est de 2.6 Alexa 647 par anticorps.

EXEMPLE 3 : Mesure de TR-FRET

Le signal de TR-FRET est mesuré dans des puits contenant 50 000 ou 100 000 cellules par puits, lesdites cellules contenant les constructions moléculaires décrites dans l'exemple 1, dans un volume final de 100 μl auxquels sont ajoutés les réactifs suivants (Figure 2) :

1A : Cellules PRK6 + anticorps anti-FLAG couplé à un cryptate de terbium (ci-après "anti-FLAG-cryptate de Terbium ") 1nM final + anticorps anti-HA couplé à l'Alexa 647 (ci-après " nanti HA-A647 ") 3nM final ;
1B : Cellules PRK6 exprimant HA-GB1 et FLAG-GB2 + anti-FLAG-cryptate de Terbium 1nM final ;
1C : Cellules PRK6 exprimant HA-GB1 et FLAG-GB2 + anti-FLAG-cryptate de Terbium 1nM final + anti HA-A647 3nM final ;
2A : Cellules PRK6 + anti-FLAG-cryptate de Terbium 1nM final + anti HA-A647 3nM final ;
2B : Cellules PRK6 exprimant HA-GB1 et FLAG-GB2-YFP + anti-FLAG-cryptate de Terbium 1nM final ;
2C : Cellules PRK6 exprimant HA-GB1, FLAG-GB2-YFP + anti-FLAG-cryptate de Terbium 1nM final + anti HA-A647 3nM final.

Pour chaque expérience, les puits ont été mis à incuber 20H à 4°C avant de mesurer la fluorescence émise.
Les conditions 1C et 2C permettent de savoir si l'expression du dimère est à peu près la même dans les 2 conditions de test. Les conditions 2B et 2C permettent de mettre en évidence un FRET éventuel entre le terbium et YFP. 1A, 1B et 2A sont les contrôles de l'expérience

EXEMPLE 4 : Mesure d'un FRET "extracellulaire", entre le cryptate de terbium et A647

Après incubations des cellules dans les conditions 1A, 1B, 2B, 2A, 1C, 2C décrites dans l'exemple 3, la fluorescence émise par les puits est mesurée sur Analyst (Molecular Devices), mode TRF Digital, délai 50μs, intégration 400μs, Excitation 330nm (filtre 330/80), Dichroïque BBUV, Emission : détection à 490nm pour Terbium, et 682nm pour A647. Le signal émis par le composé accepteur est corrigé par celui émis par le composé donneur en effectuant un ratio des signaux mesurés.
Les résultats présentés sur la figure 3 montrent une augmentation du ratio (R) avec le dimère GB1/GB2 en présence de terbium et d'A647, correspondant à une valeur de delta F de 658%, delta F étant calculé comme suit :

$$\text{Delta F} = \frac{R_{682/490}\,(\text{échantillon}) - R_{682/490}\,(\text{contrôle})}{R_{682/490}\,(\text{contrôle})}$$

Cette expérience montre qu'un FRET extracellulaire a lieu quelles que soient les constructions moléculaires utilisées, même si l'on observe une légère diminution du signal lorsque le dimère comporte une YFP (delta F=461%). Ceci peut s'expliquer soit par une légère différence du niveau d'expression des récepteurs soit par la présence d'une compétition entre les FRET terbium/A647 et terbium/YFP
Des résultats similaires sont obtenus sur des puits contenant 100 000 cellules ou lorsque le cryptate de terbium est remplacé par un cryptate de terbium.

EXEMPLE 5 : Mesure d'un FRET transmembranaire, entre le cryptate de terbium et l'YFP.

Après incubations de cellules dans les conditions 1A, 1B, 2B, 2A, 1C, 2C décrites dans l'exemple 3, la fluorescence émise par les puits est mesurée sur Analyst (Molecular Devices), mode TRF Digital, délai 50$\mu$s, intégration 400$\mu$s, Excitation 330nm (filtre 330/80), Dichroïque BBUV, Emission : détection à 490nm pour Terbium, et 520nm pour YFP. Le signal émis par le composé accepteur est corrigé par celui émis par le composé donneur en effectuant un ratio des signaux mesurés.
Les résultats présentés dans la figure 4 montrent une augmentation du ratio observé avec le dimère GB1/GB2 en présence de terbium et YFP correspondant à une valeur de delta F comprise entre 37 et 40%.
Cette expérience montre pour la première fois l'existence d'un TR-FRET transmembranaire, entre le cryptate de terbium situé sur la face externe de la membrane plasmique et l'YFP située sur la face interne de cette membrane.
Des résultats similaires sont obtenus sur des puits contenant 100 000 cellules ou lorsque le cryptate de terbium est remplacé par un cryptate de terbium.

LISTE DES DOCUMENTS CITES DANS LA DESCRIPTION.

1. D. Maurel et al., Analytical Biochemistry 329(2004), 253-262 : "Cell surface detection of membrane protein interaction with homogeneous time-resolved fluorescence resonance energy transfer technology."
2. J.P. Pin et al., Biochemical Pharmacology 68(2004), 1565-1572 : "Activation mechanism of the heterodimeric GABAB receptor."
3. J. Liu et al., The Journal of Biological Chemistry, vol. 279(2004), n°16, 15824-15830 : "Molecular Determinants Involved in the Allosteric Control of Agonist Affinity in the GABAB Receptor by the GABAB2 Subunit."
4. Tsien et al., (1997), Brevet US 5 661 035.
5. Gonzalez et al., Chemistry and Biology, (1997), 4, 269-277 : "Improved fluorescent indicators of cell membrane potential that use fluorescence resonnance energy transfer."
6. Gonzalez et al., DDT, (1999), 4,9, 431-439 : "Cell-based assays and instrumentation for screening ion-channels targets."
7. Chollet et Al., J of Computer-Aided Molecular Design, (1999), 13, 209-219 : "Biophysical approaches to G protein-coupled receptors : structure, function and dynamics."
8. Turcatti et al., J Biological Chemistry, (1996), 271,33,19991-19998 : "Probing the structure and function of the Tachykinin Neurokinin-2 Receptor through biosynthetic incorporation of fluorescent amino acid at specific sites."
9. Chan et al., Cytometry, (2001), 44, 361-368 : "Fluorescence resonance energy transfer analysis of cell surface receptor interactions and signaling using spectral variants of the green fluorescent protein."
10. Wilson et al., J Biological Chemistry, (2002), 277,5,3666-3672: "Fluorescence resonance energy transfer study on the interaction between the lactate transporter MCT1 and CD147 provide information on the topology and stoichiometry of the complex in situ."
11. Guignet et al., Nature Biotechnology, (2004), 4,440-444 : "Réversible site-selective labeling of membrane proteins in live cells."

## Revendications

1. Procédé pour la mise en évidence d'un processus biologique par mesure d'un FRET, **caractérisé en ce qu'**il

comprend les étapes suivantes :

- incorporation, dans un milieu de mesure contenant une membrane lipidique, d'une entité biologique X couplée avec un premier membre d'un couple de partenaires de FRET et d'une deuxième entité biologique Y couplée avec le deuxième membre du couple de partenaires de FRET, ledit couple de partenaires de FRET étant composé d'un fluorophore donneur et d'un fluorophore accepteur, le fluorophore donneur ayant une durée de vie comprise entre 100ns et 5000μs, de préférence comprise entre 100 et 3000μs, et les membres dudit couple de partenaires de FRET étant situés de part et d'autre de la membrane lipidique ;
- excitation du milieu de mesure à la longueur d'onde d'excitation du membre donneur d'énergie ;
- mesure du signal de FRET ou des variations dudit signal émis dans ledit milieu de culture.

2. Procédé selon la revendication 1, **caractérisé en ce que** les entités biologiques sont choisies parmi les protéines membranaires intrinsèques ou extrinsèques, les protéines transmembranaires, des composés cytosolubles, des ligands à tester, des anticorps.

3. Procédé selon l'une des revendications 1 ou 2, **caractérisé en ce que** la membrane lipidique est une membrane biologique choisie parmi : la membrane plasmique, la membrane du réticulum endoplasmique, la bi-membrane mitochondriale, une membrane lysosomiale, la membrane nucléaire, la membrane d'une vésicule golgienne.

4. Procédé selon l'une quelconque des revendications 1 à 3, **caractérisé en ce qu'**il comprend une étape de stimulation du milieu de mesure, choisie parmi une stimulation chimique, thermique, électrique ou mécanique.

5. Procédé selon la revendication 4, caractérisé ce que la stimulation est une stimulation chimique consistant à rajouter dans le milieu de mesure un composé chimique à tester, et en ce que le signal de FRET est mesuré en présence et en l'absence de ce composé à tester.

6. Procédé selon la revendication 5, **caractérisé en ce que** le processus biologique est le changement de conformation d'un récepteur transmembranaire, **en ce que** les entités biologiques X et Y sont un seul et même récepteur transmembranaire marqué de part et d'autre de la membrane par le fluorophore donneur et le fluorophore accepteur, **en ce que** le composé à tester est un composé susceptible de se fixer sur ledit récepteur transmembranaire, la variation du signal de FRET étant indicative d'une modification de la conformation dudit récepteur en présence dudit composé à tester.

7. Procédé selon la revendication 1, **caractérisé en ce que** les entités biologiques X et Y sont des protéines transmembranaires, **en ce que** le processus biologique est une variation de l'interaction entre ces deux protéines, la variation du signal de FRET étant indicative d'une variation de interaction entre lesdites protéines.

8. Procédé selon la revendication 7, **caractérisé en ce que** lesdites protéines transmembranaires X et Y sont les mêmes protéines, l'une d'elles étant marquée par le fluorophore donneur et l'autre par le fluorophore accepteur, les fluorophores donneur et accepteur se trouvant de part et d'autre de ladite membrane, la variation du signal de FRET étant significative d'un phénomène d'homodimérisation.

9. Procédé selon la revendication 8, **caractérisé en ce que** l'interaction mesurée est l'homodimérisation du récepteur $GABA_B$.

10. Procédé selon la revendication 8, **caractérisé en ce que** l'interaction mesurée est une hétérodimérisation.

11. Procédé selon l'une quelconque des revendications 7 à 10, **caractérisé en ce que** l'on ajoute au milieu de mesure un ligand à tester susceptible d'affecter la dimérisation desdites protéines transmembranaires, et **en ce que** l'on mesure le signal de FRET en présence et en l'absence dudit ligand à tester.

12. Procédé selon la revendication 1, **caractérisé en ce que** l'entité biologique X est un composé cytosoluble, **en ce que** l'entité biologique Y est une protéine membranaire intrinsèque ou extrinsèque, la variation du signal de FRET étant indicative d'un phénomène de translocation dudit composé cytosoluble vers ou à partir de ladite protéine membranaire.

13. Procédé selon la revendication 12, **caractérisé en ce que** l'on ajoute au milieu de mesure un composé à tester susceptible d'affecter la translocation dudit composé cytosoluble vers ou à partir de ladite protéine membranaire,

et **en ce que** l'on mesure le signal de FRET en présence et en l'absence dudit composé à tester.

**14.** Procédé selon l'une quelconque des revendications 12 à 13, **caractérisé en ce que** la protéine membranaire est un récepteur transmembranaire et **en ce que** le composé cytosoluble est l'arrestine ou une protéine G.

**15.** Procédé selon la revendication 1, **caractérisé en ce que** l'entité biologique X est un récepteur transmembranaire situé sur la membrane plasmique et marqué dans sa partie intracellulaire par l'un des membres du couple de fluorophores donneur / accepteur, **en ce que** l'entité biologique Y est un ligand potentiel dudit récepteur transmembranaire faisant partie d'une banque de composés à tester, et marqué par l'autre membre du couple de fluorophores donneur / accepteur, la variation du signal de FRET en présence ou en l'absence dudit composé Y étant indicative de la liaison de Y audit récepteur transmembranaire.

**16.** Procédé selon la revendication 1, **caractérisé en ce que** l'entité biologique X est un récepteur transmembranaire situé sur la membrane plasmique et marqué dans sa partie intracellulaire par l'un des membres du couple de fluorophores donneur / accepteur, **en ce que** l'entité biologique Y est un ligand connu dudit récepteur transmembranaire, et marqué par l'autre membre du couple de fluorophores donneur / accepteur, et **en ce que** l'on ajoute au milieu de mesure un composé à tester, la variation du signal de FRET en présence ou en l'absence dudit composé à tester étant indicative de la liaison dudit composé à tester audit récepteur transmembranaire X.

**17.** Procédé selon l'une quelconque des revendications 1 à 16, **caractérisé en ce que** le fluorophore donneur est un chélate de terre rare ou un cryptate de terre rare, la terre rare étant de préférence choisie parmi l'europium et le terbium.

**18.** Procédé selon la revendication 17, **caractérisé en ce que** le fluorophore donneur est un cryptate de terre rare comportant un motif pyridine.

**19.** Procédé selon la revendication 18, **caractérisé en ce que** le fluorophore donneur est un cryptate ou chélate de terbium et **en ce que** le fluorophore accepteur est une protéine fluorescente.

**20.** Procédé selon l'une quelconque des revendications 1 à 18, **caractérisé en ce que** le fluorophore accepteur est choisi parmi les rhodamines, les cyanines, les squaraines, les BODIPYs, les fluorescéines, les composés de la famille des AlexaFluors, les quantum dots, les phycobiliprotéines telles que la B-phycoérythrine, la R-phycoérythrine, la C-phycocyanine, l'allophycocyanine, la GFP et ses dérivés, l'YFP, la CFP, une protéine fluorescente de corail.

**21.** Procédé selon l'une quelconque des revendications 1 à 20, **caractérisé en ce que** le couplage des entités biologiques X ou Y avec les fluorophores donneur et accepteur est choisi parmi : un couplage par liaison covalente ; un couplage indirect par un système biotine / streptavidine, tag / anticorps anti-tag, choisi parmi les systèmes 6HIS/anti-6HIS, , FLAG/anti-FLAG, DNP/anti-DNP, GST/anti-GST, c-myc/anti-c-myc, HA/anti-HA, un couplage via un anticorps spécifique de l'entité X ou Y à marquer.

## Patentansprüche

**1.** Verfahren zum Nachweis eines biologischen Prozesses durch Messung eines FRET, **dadurch gekennzeichnet, dass** es die folgenden Schritte umfasst:

- Vermischen in einem Messmedium, das eine Lipidmembran enthält, einer biologischen Einheit X, die an ein erstes Element eines Paars von FRET-Partnern gekoppelt ist und einer zweiten biologischen Einheit Y, die an das zweite Element des Paares von FRET-Partnern gekoppelt ist, wobei das Paar von FRET-Partnern aus einem Donor-Fluorophor und einem Akzeptor-Fluorophor besteht, wobei das Donor-Fluorophor eine Lebensdauer zwischen 100 ns und 5000 μs aufweist, vorzugsweise zwischen 100 und 3000 μs, und sich die Elemente des Paares von FRET-Partnern auf beiden Seiten der Lipidmembran befinden,
- Anregen des Messmediums bei der Anregungswellenlänge des Energiedonorelements,
- Messen des FRET-Signals oder von Variationen des Signals, die in dem Kulturmedium emittiert werden.

**2.** Verfahren gemäß Anspruch 1, **dadurch gekennzeichnet, dass** die biologischen Einheiten ausgewählt werden aus intrinsischen oder extrinsischen Membranproteinen, Transmembranproteinen, zelllöslichen Verbindungen, Testliganden, Antikörpern.

**3.** Verfahren gemäß einem der Ansprüche 1 oder 2, **dadurch gekennzeichnet, dass** die Lipidmembran eine biologische Membran ist, die ausgewählt wird aus: der Plasmamembran, der Membran des endoplasmatischen Retikulums, der mitochondrialen Doppelmembran, einer lysosomalen Membran, der Kernmembran, der Membran eines Golgivesikels.

**4.** Verfahren gemäß einem der Ansprüche 1 bis 3, **dadurch gekennzeichnet, dass** es einen Schritt der Stimulation des Messmediums umfasst, die ausgewählt wird aus chemischer, thermischer, elektrischer oder mechanischer Stimulation.

**5.** Verfahren gemäß Anspruch 4, **dadurch gekennzeichnet, dass** die Stimulation eine chemische Stimulation ist, die aus dem Hinzufügen einer chemischen Testverbindung in das Messmedium besteht und dass das FRET-Signal in Gegenwart und Abwesenheit dieser Testverbindung gemessen wird.

**6.** Verfahren gemäß Anspruch 5, **dadurch gekennzeichnet, dass** der biologische Prozess die Konformationsänderung eines Transmembranrezeptors ist, dass die biologischen Einheiten X und Y ein einziger und identischer Transmembranrezeptor sind, der auf beiden Seiten der Membran vom Donor-Fluorophor und vom Akzeptor-Fluorophor markiert ist, dass die Testverbindung eine Verbindung ist, die dazu in der Lage ist, sich an den Transmembranrezeptor zu binden, wobei die Variation des FRET-Signals eine Änderung der Konformation des Rezeptors in Anwesenheit der Testverbindung anzeigt.

**7.** Verfahren gemäß Anspruch 1, **dadurch gekennzeichnet, dass** die biologischen Einheiten X und Y Transmembranproteine sind, dass der biologische Prozess eine Variation der Wechselwirkung zwischen diesen beiden Proteinen ist, wobei die Variation des FRET-Signals eine Variation der Wechselwirkung zwischen den Proteinen anzeigt.

**8.** Verfahren gemäß Anspruch 7, **dadurch gekennzeichnet, dass** die Transmembranproteine X und Y dieselben Proteine sind, von denen eines mit dem Donor-Fluorophor und das andere mit dem Akzeptor-Fluorophor markiert ist, wobei sich die Donor- und Akzeptor-Fluorophore auf beiden Seiten der Membran befinden, wobei die Variation des FRET-Signals ein Homodimerisierungsphänomen anzeigt.

**9.** Verfahren gemäß Anspruch 8, **dadurch gekennzeichnet, dass** die gemessene Wechselwirkung die Homodimerisierung des $GABA_B$ Rezeptors ist.

**10.** Verfahren gemäß Anspruch 8, **dadurch gekennzeichnet, dass** die gemessene Wechselwirkung eine Heterodimerisierung ist.

**11.** Verfahren gemäß einem der Ansprüche 7 bis 10, **dadurch gekennzeichnet, dass** dem Messmedium ein Testligand hinzugefügt wird, der dazu in der Lage ist, die Dimerisierung der Transmembranproteine zu bewirken, und dass das FRET-Signal in Gegenwart und Abwesenheit des Testliganden gemessen wird.

**12.** Verfahren gemäß Anspruch 1, **dadurch gekennzeichnet, dass** die biologische Einheit X eine zelllösliche Verbindung ist, dass die biologische Einheit Y ein intrinsisches oder extrinsisches Membranprotein ist, wobei die Variation des FRET-Signals ein Translokationsphänomen der zelllöslichen Verbindung zu oder von dem Membranprotein anzeigt.

**13.** Verfahren gemäß Anspruch 12, **dadurch gekennzeichnet, dass** dem Messmedium eine Testverbindung hinzugefügt wird, die dazu in der Lage ist, die Translokation der zelllöslichen Verbindung zu oder von dem Membranprotein zu bewirken, und dass das FRET-Signal in Gegenwart und Abwesenheit der Testverbindung gemessen wird.

**14.** Verfahren gemäß einem der Ansprüche 12 bis 13, **dadurch gekennzeichnet, dass** das Membranprotein ein Transmembranrezeptor ist und dass die zelllösliche Verbindung Arrestin oder ein G-Protein ist.

**15.** Verfahren gemäß Anspruch 1, **dadurch gekennzeichnet, dass** die biologische Einheit X ein Transmembranrezeptor ist, der sich auf der Plasmamembran befindet und an seinem intrazellulären Teil mit einem der Elemente des Donor-/Akzeptor-Fluorophor -Paars markiert ist, und dass die biologische Einheit Y ein potentieller Ligand des Transmembranrezeptors ist, der Teil einer Bibliothek von Testverbindungen ist und mit dem anderen Element des Donor-/Akzeptor-Fluorophor-Paars markiert ist, wobei die Variation des FRET-Signals in Gegenwart oder Abwesenheit der Verbindung Y die Bindung von Y an den Transmembranrezeptor anzeigt.

**16.** Verfahren gemäß Anspruch 1, **dadurch gekennzeichnet, dass** die biologische Einheit X ein Transmembranrezeptor ist, der sich auf der Plasmamembran befindet und an seinem intrazellulären Teil mit einem der Elemente des Donor-/Akzeptor-Fluorophor-Paars markiert ist, dass die biologische Einheit Y ein bekannter Ligand des Transmembranrezeptors ist und mit dem anderen Element des Fluorophor Donor-/Akzeptor-Paars markiert ist, und dass dem Messmedium eine Testverbindung hinzugefügt wird, wobei die Variation des FRET-Signals in Gegenwart oder in Abwesenheit der Testverbindung die Bindung der Testverbindung an den Transmembranrezeptor X anzeigt.

**17.** Verfahren gemäß einem der Ansprüche 1 bis 16, **dadurch gekennzeichnet, dass** das Donor-Fluorophor ein Chelat einer Seltenen Erde oder ein Kryptat einer Seltenen Erde ist, wobei die Seltene Erde vorzugsweise ausgewählt wird aus Europium und Terbium.

**18.** Verfahren gemäß Anspruch 17, **dadurch gekennzeichnet, dass** das Donor-Fluorophor ein Kryptat einer seltenen Erde ist, das eine Pyridin-Einheit umfasst.

**19.** Verfahren gemäß Anspruch 18, **dadurch gekennzeichnet, dass** das Donor-Fluorophor ein Kryptat oder Chelat von Terbium ist und dass das Akzeptor-Fluorophor ein fluoreszierendes Protein ist.

**20.** Verfahren gemäß einem der Ansprüche 1 bis 18, **dadurch gekennzeichnet, dass** das Akzeptor-Fluorophor ausgewählt wird aus Rhodaminen, Cyaninen, Squarainen, BODIPYs, Fluoresceinen, Verbindungen der AlexaFluor-Familie, Quantenpunkten, Phycobiliproteinen, wie etwa B-Phycoerythrin, R-Phycoerythrin, C-Phycocyanin, Allophycocyanin, GFP und deren Derivaten, YFP, CFP, einem fluoreszierenden Korallenprotein.

**21.** Verfahren gemäß einem der Ansprüche 1 bis 20, **dadurch gekennzeichnet, dass** die Kopplung der biologischen Einheiten X oder Y mit den Donor- und Akzeptor-Fluorophoren ausgewählt wird aus: einer Kupplung durch kovalente Bindung, einer indirekten Kopplung über ein System Biotin/Streptavidin, Tag/Anti-Tag-Antikörper, ausgewählt aus den Systemen 6HIS/Anti-6HIS, FLAG/Anti-FLAG, DNP/Anti-DNP, GST/Anti-GST, c-myc/Anti-c-myc, HA/Anti-HA, einer Kopplung über einen Antikörper, der spezifisch für die zu markierende Einheit X oder Y ist.

## Claims

**1.** A method of revealing a biological process using a FRET measurement, **characterized in that** it comprises the following steps:

- incorporating, into a measurement medium containing a lipid membrane, a biological entity X coupled with a first member of a pair of FRET partners and a second biological entity Y coupled with the second member of the pair of FRET partners, the pair of FRET partners being composed of a donor fluorophor and an acceptor fluorophor, the donor fluorophor having a lifetime of between 100 ns and 5000 $\mu$s, preferably of between 100 and 3000 $\mu$s, and the members of said pair of FRET partners being located on either side of the lipid membrane;
- exciting the measurement medium at the excitation wavelength of the energy-donating member; and
- measuring the FRET signal or the variations in said signal emitted in said culture medium.

**2.** The method according to claim 1, **characterized in that** the biological entities are selected from intrinsic or extrinsic membrane proteins, transmembrane proteins, cytosoluble compounds, test ligands and antibodies.

**3.** The method according to claim 1 or 2, **characterized in that** the lipid membrane is a biological membrane selected from the plasma membrane, the endoplasmic reticulum membrane, the mitochondrial double membrane, a lysosomal membrane, the nuclear membrane and the Golgi vesicle membrane.

**4.** The method according to any one of claims 1 to 3, **characterized in that** it comprises a step for stimulating the measurement medium, selected from chemical, thermal, electrical and mechanical stimulation.

**5.** The method according to claim 4, **characterized in that** the stimulation is a chemical stimulation that consists in adding a chemical test compound to the measurement medium, and **in that** the FRET signal is measured in the presence and absence of this test compound.

**6.** The method according to claim 5, **characterized in that** the biological process is the conformational change of a transmembrane receptor, **in that** the biological entities X and Y are one and the same transmembrane receptor

labeled on either side of the membrane with the donor fluorophor and the acceptor fluorophor, and **in that** the test compound is a compound capable of binding to said transmembrane receptor, the variation in the FRET signal being indicative of a modification of the conformation of said receptor in the presence of said test compound.

**7.** The method according to claim 1, **characterized in that** the biological entities X and Y are transmembrane proteins and **in that** the biological process is a variation in the interaction between these two proteins, the variation in the FRET signal being indicative of a variation in interaction between said proteins.

**8.** The method according to claim 7, **characterized in that** said transmembrane proteins X and Y are the same proteins, one of them being labeled with the donor fluorophor and the other with the acceptor fluorophor, the donor and acceptor fluorophors being located on either side of said membrane, the variation in the FRET signal being indicative of a homodimerization phenomenon.

**9.** The method according to claim 8, **characterized in that** the measured interaction is the homodimerization of the $GABA_B$ receptor.

**10.** The method according to claim 8, **characterized in that** the measured interaction is a heterodimerization.

**11.** The method according to any one of claims 7 to 10, **characterized in that** a test ligand capable of affecting the dimerization of said transmembrane proteins is added to the measurement medium, and **in that** the FRET signal is measured in the presence and absence of said test ligand.

**12.** The method according to claim 1, **characterized in that** the biological entity X is a cytosoluble compound and **in that** the biological entity Y is an intrinsic or extrinsic membrane protein, the variation in the FRET signal being indicative of a phenomenon of translocation of said cytosoluble compound to or from said membrane protein.

**13.** The method according to claim 12, **characterized in that** a test compound capable of affecting the translocation of said cytosoluble compound to or from said membrane protein is added to the measurement medium, and **in that** the FRET signal is measured in the presence and absence of said test compound.

**14.** The method according to any one of claims 12 and 13, **characterized in that** the membrane protein is a transmembrane receptor and **in that** the cytosoluble compound is arrestin or a G protein.

**15.** The method according to claim 1, **characterized in that** the biological entity X is a transmembrane receptor located on the plasma membrane and labeled in its intracellular part with one member of the pair of donor/acceptor fluorophors, and **in that** the biological entity Y is a potential ligand for said transmembrane receptor which forms part of a bank of test compounds and is labeled with the other member of the pair of donor/acceptor fluorophors, the variation in the FRET signal in the presence or absence of said compound Y being indicative of the binding of Y to said transmembrane receptor.0

**16.** The method according to claim 1, **characterized in that** the biological entity X is a transmembrane receptor located on the plasma membrane and labeled in its intracellular part with one member of the pair of donor/acceptor fluorophors, **in that** the biological entity Y is a known ligand for said transmembrane receptor which is labeled with the other member of the pair of donor/acceptor fluorophors, and **in that** a test compound is added to the measurement medium, the variation in the FRET signal in the presence or absence of said test compound being indicative of the binding of said test compound to said transmembrane receptor X.

**17.** The method according to any one of claims 1 to 16, **characterized in that** the donor fluorophor is a rare earth chelate or a rare earth cryptate, the rare earth preferably being selected from europium and terbium.

**18.** The method according to claim 17, **characterized in that** the donor fluorophor is a rare earth cryptate containing a pyridine unit.

**19.** The method according to claim 18, **characterized in that** the donor fluorophor is a terbium cryptate or chelate and **in that** the acceptor fluorophor is a fluorescent protein.

**20.** The method according to any one of claims 1 to 18, **characterized in that** the acceptor fluorophor is selected from rhodamines, cyanins, squaraines, BODIPYs, fluoresceins, compounds of the AlexFluor family, quantum dots, phycobiliproteins such as B-phycoerythrin, R-phycoerythrin, C-phycocyanin and allophycocyanin, GFP and its derivatives, YFP, CFP and a fluorescent coral protein.

**21.** The method according to any one of claims 1 to 20, **characterized in that** the coupling of the biological entity X or Y with the donor and acceptor fluorophors is selected from the following: coupling by covalent bonding; indirect coupling by a biotin/streptavidin system, a tag/anti-tag antibody system selected from 6HIS/anti-6HIS, FLAG/anti-FLAG, DNP/anti-DNP, GST/anti-GST, c-myc/anti-c-myc and HA/anti-HA systems; and coupling via an antibody specific for the entity X or Y to be labeled.

HA-GB1+FLAG-GB2 :

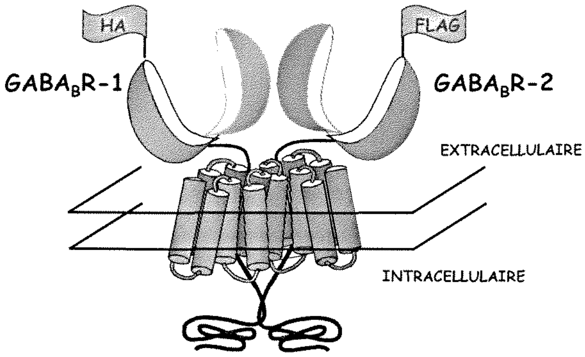


HA-GB1+FLAG-GB2-YFP :

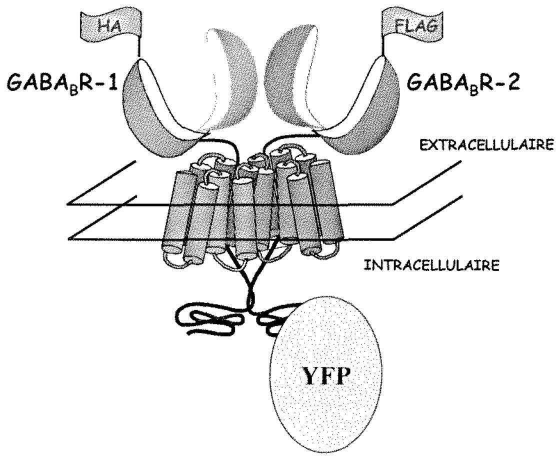


FIG.1

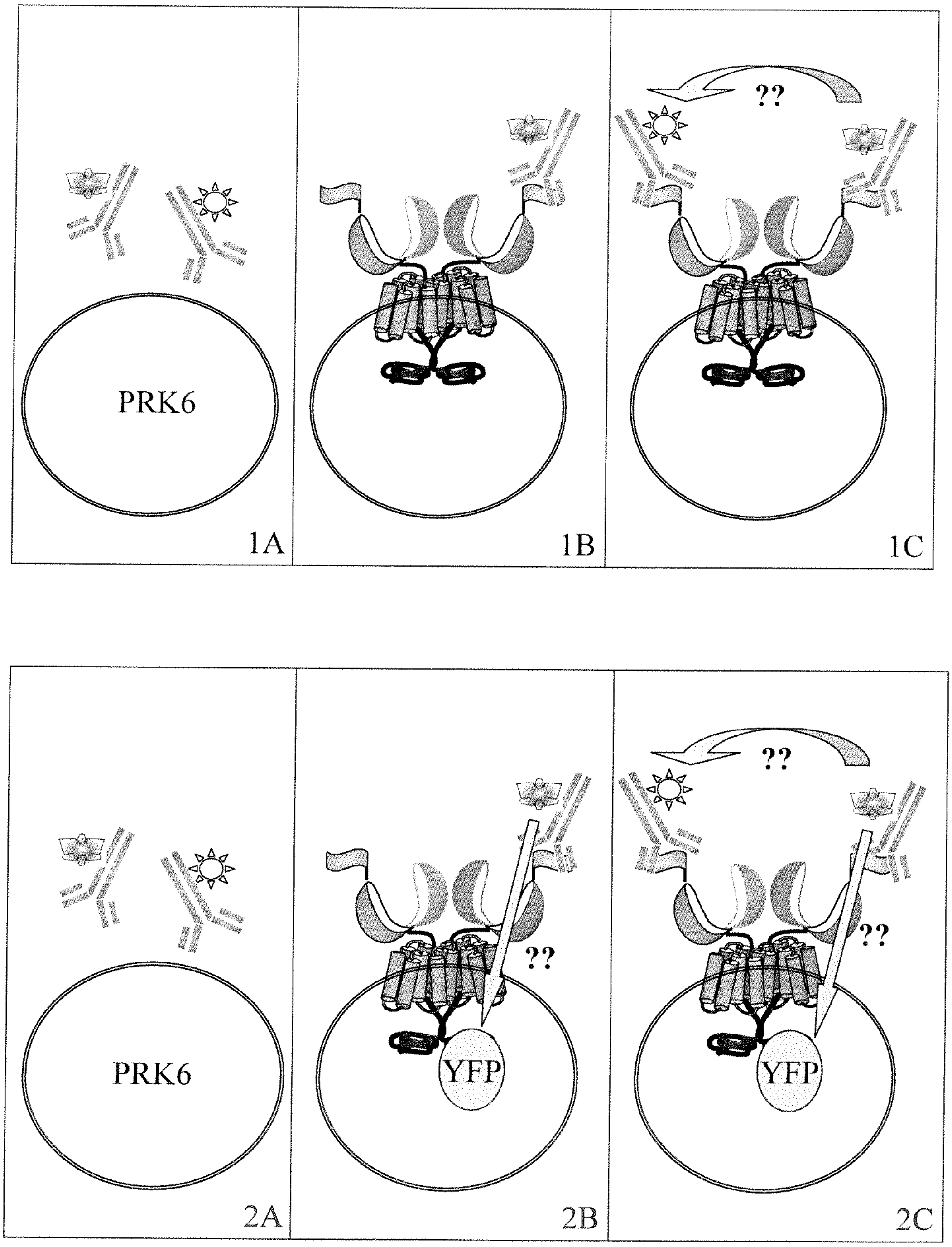


FIG. 2

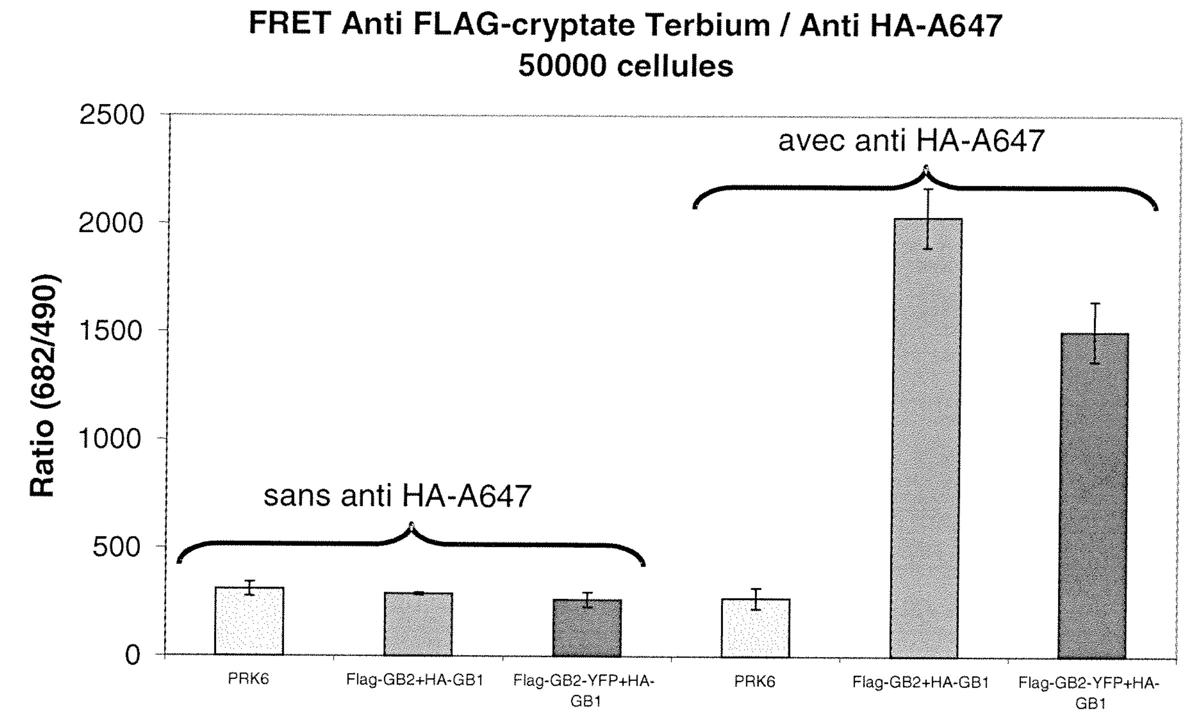
FRET Anti FLAG-cryptate Terbium / Anti HA-A647
50000 cellules
Ratio (682/490)
2500
2000
1500
1000
500
0
avec anti HA-A647
sans anti HA-A647
PRK6
Flag-GB2+HA-GB1
Flag-GB2-YFP+HA-GB1
PRK6
Flag-GB2+HA-GB1
Flag-GB2-YFP+HA-GB1

**FIG. 3**

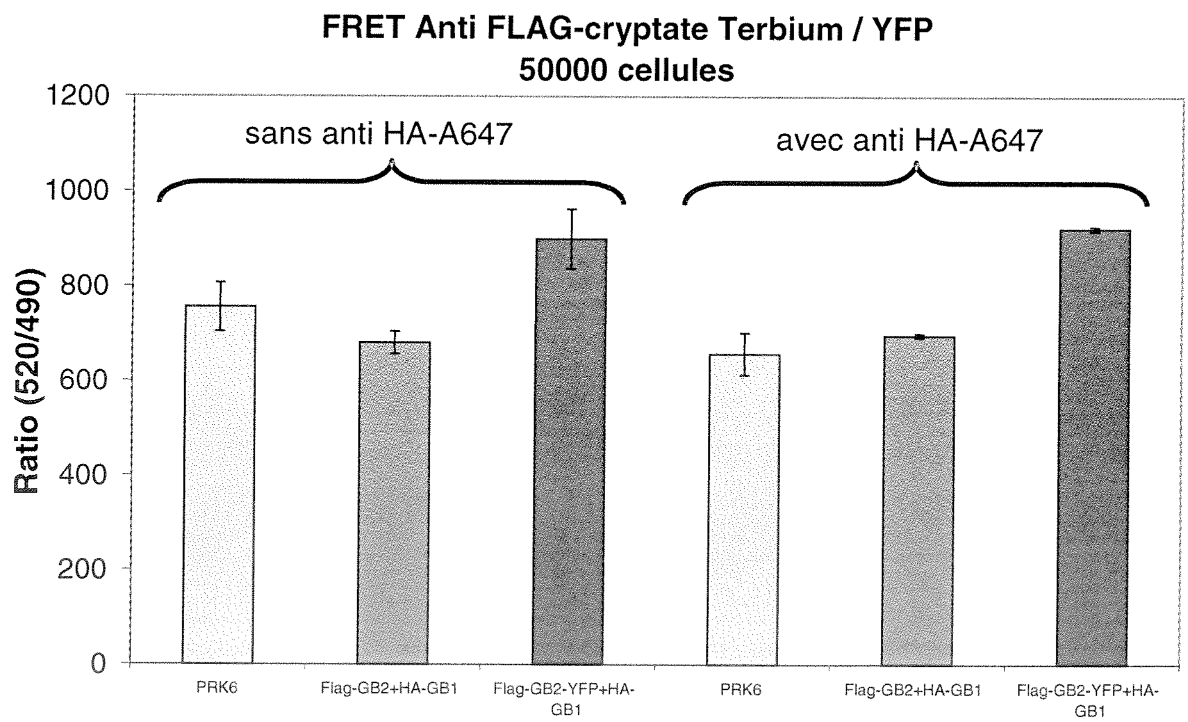
FRET Anti FLAG-cryptate Terbium / YFP
50000 cellules
Ratio (520/490)
1200
1000
800
600
400
200
0
sans anti HA-A647
avec anti HA-A647
PRK6
Flag-GB2+HA-GB1
Flag-GB2-YFP+HA-GB1
PRK6
Flag-GB2+HA-GB1
Flag-GB2-YFP+HA-GB1

**FIG. 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03005028 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 403593 A
- US 5324825 A
- US 5202423 A
- US 5316909 A
- EP 0180492 A
- EP 0601113 A
- WO 0196877 A
- WO 02083937 A
- US 5661035 A, Tsien


### Littérature non-brevet citée dans la description


- Homogeneous time resolved fluorescence energy transfer using rare earth cryptates as a tool for probing molecular interactions in biology. *Spectrochimica Acta Part A,* 2001, vol. 57, 2197-2211
- **G.T. HERMANSON.** Bioconjugate Techniques. Academic Press, 1996
- **MATHIS G.** Rare Earth Cryptates and Homogeneous Fluoroimmunoassays with Human Sera. *Clin Chem,* 1993, vol. 39 (9), 1953-1959
- **SAMBROOK et al.** Molecular Cloning : A Laboratory Manual. Cold Spring Harbor Laboratory, 1989
- **MEHIER-HUMBERT S ; GUY RH.** Physical methods for gene transfer: improving the kinetics of gene delivery into cells. *Advanced Drug Delivery Reviews,* 2005, vol. 57 (5), 733-753
- *Methods,* Juin 2004, vol. 33 (2), 93-186
- **LAKOWICZ.** Principles of fluorescence spectroscopy. Kluwer academic/plenum publishers, 1999
- *Journal of Biological Chemistry,* 26 Juin 1998, vol. 273 (26), 15887-15890
- *J. Am. Chem. Soc.,* 2003, vol. 125, 7180-7181
- **PARMENTIER ML ; PREZEAU L ; BOCKAERT J ; PIN JP.** A model for the functioning of family 3 GPCRs. *Trends Pharmacol Sci.,* 2002, vol. 23 (6), 268-74
- **BISSANTZ C.** Conformational changes of G protein-coupled receptors during their activation by agonist binding. *J Recept Signal Transduct Res.,* 2003, vol. 23 (2-3), 123-53
- **PRADO MA ; EVANS-BAIN B ; DICKERSON IM.** Receptor component protein (RCP): a member of a multi-protein complex required for G-protein-coupled signal transduction. *Biochem Soc Trans.,* 2002, vol. 30 (4), 460-4
- **SEBASTIEN BULENGER ; STEFANO MARULLO ; MICHEL BOUVIER.** Emerging role of homo- and hetero-dimerization in G-protein coupled receptor biosynthesis and maturation. *Trends in Pharmacological Sciences,* 2005, vol. 26 (3), 131-37
- **HANSEN JL ; SHEIKH SP.** Functional consequences of 7TM receptor dimerization. *Eur J Pharm Sci.,* 2004, vol. 23 (4-5), 301-17
- **VASSART G ; PARDO L ; COSTAGLIOLA S.** A molecular dissection of the glycoprotein hormone receptors. *Trends Biochem Sci.,* 2004, vol. 29 (3), 119-26
- **MILLIGAN G.** G protein-coupled receptor dimerization: function and ligand pharmacology. *Mol Pharmacol.,* 2004, vol. 66 (1), 1-7
- **TERRILLON, S. ; BOUVIER, M.** Roles of G-protein-coupled receptor dimerization. *EMBO Rep.,* 2004, vol. 5, 30-34
- **RIOS CD ; JORDAN BA ; GOMES I ; DEVI LA.** G-protein-coupled receptor dimerization: modulation of receptor function. *Pharmacol Ther.,* 2001, vol. 92 (2-3), 71-87
- **D. MAUREL et al.** Cell surface detection of membrane protein interaction with homogeneous time-resolved fluorescence resonance energy transfer technology. *Analytical Biochemistry,* 2004, vol. 329, 253-262

- **J.P. PIN et al.** Activation mechanism of the heterodimeric GABAB receptor. *Biochemical Pharmacology,* 2004, vol. 68, 1565-1572
- **J. LIU et al.** Molecular Determinants Involved in the Allosteric Control of Agonist Affinity in the GABAB Receptor by the GABAB2 Subunit. *The Journal of Biological Chemistry,* 2004, vol. 279 (16), 15824-15830
- **GONZALEZ et al.** Improved fluorescent indicators of cell membrane potential that use fluorescence resonnance energy transfer. *Chemistry and Biology,* 1997, vol. 4, 269-277
- **GONZALEZ et al.** Cell-based assays and instrumentation for screening ion-channels targets. *DDT,* 1999, vol. 4 (9), 431-439
- **CHOLLET et al.** Biophysical approaches to G protein-coupled receptors : structure, function and dynamics. *J of Computer-Aided Molecular Design,* 1999, vol. 13, 209-219
- **TURCATTI et al.** Probing the structure and function of the Tachykinin Neurokinin-2 Receptor through biosynthetic incorporation of fluorescent amino acid at specific sites. *J Biological Chemistry,* 1996, vol. 271 (33), 19991-19998
- **CHAN et al.** Fluorescence resonance energy transfer analysis of cell surface receptor interactions and signaling using spectral variants of the green fluorescent protein. *Cytometry,* 2001, vol. 44, 361-368
- **WILSON et al.** Fluorescence resonance energy transfer study on the interaction between the lactate transporter MCT1 and CD147 provide information on the topology and stoichiometry of the complex in situ. *J Biological Chemistry,* 2002, vol. 277 (5), 3666-3672
- **GUIGNET et al.** Réversible site-selective labeling of membrane proteins in live cells. *Nature Biotechnology,* 2004, vol. 4, 440-444